# EUROPEAN PATENT APPLICATION

(11) **EP 4 751 586 A2**
(43) Date of publication of application: **03.06.2026**
(21) Application number: 26172322.5
(22) Date of filing: 13.03.2020
(51) Int. Cl.: A24F 1/00

(54) **SMOKING SUBSTITUTE SYSTEM**

(30) Priority: 22.03.2019 EP 19020139
(62) Divisional of application: 20715712.4
(71) Applicant: Imperial Tobacco Limited, Bristol BS3 2LL (GB)
(72) Inventor: FERRIE, Kate, Bristol, BS3 2LL (GB); SHENTON, Ross, Bristol, BS3 2LL (GB); LORD, Chris, Bristol, BS3 2LL (GB); MURRAY, Samantha, Bristol, BS3 2LL (GB); JONES, David, Bristol, BS3 2LL (GB); BENYEZZAR, Med, Bristol, BS3 2LL (GB)
(74) Representative: Mewburn Ellis LLP

(57) **Abstract**

Disclosed is a smoking substitute system having a device comprising a controller configured to receive a command to enter a safety mode and, in response, disable at least one function of the device.

## Description

### TECHNICAL FIELD

The present invention relates to a smoking substitute system and particularly, although not exclusively, to a system having a smoking substitute device and a method of operating the device.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles (also referred to as a "consumables") that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories.

There are a number of different categories of smoking substitute systems, each utilising a different smoking substitute approach.

One approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

Another approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is pre-filled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

Because smoking substitute systems involve heating a substance, they can (in some cases) present a safety risk.

There may be a need for improved design of smoking substitute systems, in particular HT smoking substitute systems, to enhance the user experience and improve the function of the HT smoking substitute system.

The present disclosure has been devised in the light of the above considerations.

### SUMMARY OF THE INVENTION

At its most general, the present invention relates to a smoking substitute device having a safety mode (e.g. a child safety mode).

According to a first aspect of the present invention, there is provided a smoking substitute device comprising a controller configured to receive a command to enter a safety mode and, in response, disable at least one function of the device.

By providing a device able to operate in a safety mode, the device may prevent inadvertent activation of functions of the device that could cause harm to e.g. a child attempting to use the device. Further, the safety mode may prevent inadvertent activation of the device in situations where such activation could be dangerous (e.g. in a user's pocket or during transport).

Optional features will now be set out. These are applicable singly or in any combination with any aspect.

Disabling at least one function of the device may comprise disabling the operation one or more of a heater, display, light indicator, user input, battery level indicator, and a haptic feedback module of the device. The at least one function may be disabled for a predetermined period of time, or until a command is received to leave the safety mode. The function or plurality functions that are disabled in safety mode may be configurable by a user (e.g. via interaction with a user input module of the device).

The device may comprise a user input module operatively connected to the controller. The user input module may be configured to transmit the command to enter the safety mode to the controller based an interaction by a user with the user input module. The user input module may comprise a button. The command may comprise a signal indicative of a pattern of button presses. This may, for example, be a predetermined number of successive button presses (e.g. within a predetermined time period). For example, the command may comprise a signal indicative of four, five, or six button presses within a predetermined period of time.

Similarly, the command may comprise a signal indicative of the button being depressed for a predetermined time period. For example, the command may be in the form of a signal indicative of the button being pressed for more than e.g. 2 seconds, or e.g. 4 seconds, or e.g. 5 seconds. The command may comprise a signal indicative of a pattern of button presses that includes both a plurality of successive button presses and one or more button presses for a predetermined period of time.

Alternatively, the user input module may comprise a touch screen, and the command may be in the form of a numerical code entered using the touchscreen.

The device may comprise a sensor for detecting a user inhalation from the device. The sensor may be a puff sensor. The puff sensor may e.g. be a pressure sensor or an acoustic sensor. The command may comprise a signal (i.e. from the puff sensor) indicative of a pattern of user inhalation from the device. The pattern of user inhalation may comprise an inhalation for a predetermined period of time, or an inhalation having a predetermined airflow (e.g. which may be measured via pressure change). The pattern of user inhalation may comprise a series of inhalations (e.g. successive inhalations).

The command may be generated in response to an operating condition of the device. For example, the command may be generated in response to the absence of a consumable engaged with the device. That is, the device may comprise a sensor to detect the presence of a consumable engaged with the device, and may enter safety mode when a consumable is not detected.

The device may comprise a heater, a body, a cap engagable with the body for at least partly enclosing the heater, and a cap sensor for detecting whether the cap is open. The command may comprise a signal from the cap sensor indicative of the cap being open. That is, the controller may be configured to enter the safety mode when the cap of the device is open (i.e. when the cap is detected as being open).

In some forms the cap may remain engaged with the body when in the open position (e.g. the cap may be slideably between a closed position and an open position). The cap may additionally or alternatively be fully removable from the body. In this respect, the command may be a signal indicative of the cap being fully removed from the body. In some embodiments a tool may be required to remove the cap from the device (e.g. fully). The device may comprise a sensor to detect use of the tool with the cap so as to be able to provide a signal indicative of the cap being removed.

The device may comprise a temperature sensor for measuring a temperature of a portion of the device. For example, the temperature sensor may be arranged to measure a temperature of the heater or a battery of the device. The temperatures sensor may alternatively be arranged to measure an ambient (i.e. external room) temperature. The command may be in the form of a signal indicative of the measured temperature (measured by the temperature sensor) being greater than a predetermined threshold temperature.

In some embodiments the command may comprise a signal indicative of an absence of user interaction or input for a predetermined time period. Such user input may comprise e.g. button presses and/or inhalation. The predetermined time period may be e.g. 5 minutes, 15 minutes, or 20 minutes.

The controller may be configured to enable the at least one function of the device upon receipt of a leave command to leave the safety mode. The leave command may be the same as the (enter) command as described above. For example, the leave command may comprise a signal indicative of a pattern of button presses, inhalation pattern, etc. Where (safety mode entering) command comprises a signal indicative of an operating condition of the device, the leave command may comprise a signal indicative of that operating condition no longer being present. For example, where the controller enters the safety mode in response to opening of the cap, closing the cap may cause the controller to leave the safety mode.

The safety mode may be considered a child safety mode.

The device may comprise a user output module. The controller may be configured to control the user output module to provide a user output in response to entering and/or leaving the safety mode. For example, the user output module may comprise a haptic feedback module (e.g. comprising a vibration mechanism). The controller may control the haptic feed module to provide haptic feedback upon entering or leaving the safety mode. Similarly, the controller may be configured to control the user output module to provide a user output (e.g. haptic feedback) when a user attempts to activate a disabled function of the device in safety mode. Such an attempt may be in the form of an interaction with a user input component of the user input module (e.g. an inhalation from the device or a button press).

The device may comprise an elongate body. An end of the elongate body may be configured for engagement with an aerosol-forming article (e.g. a heated tobacco (HT) consumable. The device may comprise a cavity that is configured for receipt of at least a portion of the consumable (i.e. for engagement with the consumable). The aerosol-forming article may be of the type that comprises an aerosol former (e.g. carried by an aerosol-forming substrate).

As discussed above, the device may comprise a heater, which may be for heating the aerosol-forming article. The heater may comprise a heating element, which may be in the form of a rod that extends from the body of the device. The heating element may extend from the end of the body that is configured for engagement with the aerosol-forming article. The temperature sensor may be in the form of a thermocouple mounted to or forming part of the heating element.

The heater (and thus the heating element) may be rigidly mounted to the body. The heating element may be elongate so as to define a longitudinal axis and may, for example, have a transverse profile (i.e. transverse to a longitudinal axis of the heating element) that is substantially circular (i.e. the heating element may be generally cylindrical). Alternatively, the heating element may have a transverse profile that is rectangular (i.e. the heater may be a "blade heater"). The heating element may alternatively be in the shape of a tube (i.e. the heater may be a "tube heater"). The heating element may take other forms (e.g. the heating element may have an elliptical transverse profile). The shape and/or size (e.g. diameter) of the transverse profile of the heating element may be generally consistent for the entire length (or substantially the entire length) of the heating element.

The heating element may be between 15 mm and 25 mm long, e.g. between 18 mm and 20 mm long, e.g. around 19 mm long. The heating element may have a diameter of between 1.5 mm and 2.5 mm, e.g. a diameter between 2 mm and 2.3 mm, e.g. a diameter of around 2.15 mm.

The heating element may be formed of ceramic. The heating element may comprise a core (e.g. a ceramic core) comprising Al2O3. The core of the heating element may have a diameter of 1.8 mm to 2.1 mm, e.g. between 1.9 mm and 2 mm. The heating element may comprise an outer layer (e.g. an outer ceramic layer) comprising Al2O3. The thickness of the outer layer may be between 160 µm and 220 µm, e.g. between 170 µm and 190 µm, e.g. around 180 µm. The heating element may comprise a heating track, which may extend longitudinally along the heating element. The heating track may be sandwiched between the outer layer and the core of the heating element. The heating track may comprise tungsten and/or rhenium. The heating track may have a thickness of around 20 µm.

The heating element may be located in the cavity (of the device), and may extend (e.g. along a longitudinal axis) from an internal base of the cavity towards an opening of the cavity. The length of the heating element (i.e. along the longitudinal axis of the heater) may be less than the depth of the cavity. Hence, the heating element may extend for only a portion of the length of the cavity. That is, the heating element may not extend through (or beyond) the opening of the cavity.

The heating element may be configured for insertion into an aerosol-forming article (e.g. a HT consumable) when an aerosol-forming article is received in the cavity. In that respect, a distal end (i.e. distal from a base of the heating element where it is mounted to the device) of the heating element may comprise a tapered portion, which may facilitate insertion of the heating element into the aerosol-forming article. The heating element may fully penetrate an aerosol-forming article when the aerosol-forming article is received in the cavity. That is, the entire length, or substantially the entire length, of the heating element may be received in the aerosol-forming article.

The heating element may have a length that is less than, or substantially the same as, an axial length of an aerosol-forming substrate forming part of an aerosol-forming article (e.g. a HT consumable). Thus, when such an aerosol-forming article is engaged with the device, the heating element may only penetrate the aerosol-forming substrate, rather than other components of the aerosol-forming article. The heating element may penetrate the aerosol-forming substrate for substantially the entire axial length of the aerosol forming-substrate of the aerosol-forming article. Thus, heat may be transferred from (e.g. an outer circumferential surface of) the heating element to the surrounding aerosol-forming substrate, when penetrated by the heating element. That is, heat may be transferred radially outwardly (in the case of a cylindrical heating element) or e.g. radially inwardly (in the case of a tube heater).

Where the heater is a tube heater, the heating element of the tube heater may surround at least a portion of the cavity. When the portion of the aerosol-forming article is received in the cavity, the heating element may surround a portion of the aerosol-forming article (i.e. so as to heat that portion of the aerosol-forming article). In particular, the heating element may surround an aerosol forming substrate of the aerosol-forming article. That is, when an aerosol-forming article is engaged with the device, the aerosol forming substrate of the aerosol-forming article may be located adjacent an inner surface of the (tubular) heating element. When the heating element is activated, heat may be transferred radially inwardly from the inner surface of the heating element to heat the aerosol forming substrate.

The cavity may comprise a (e.g. circumferential) wall (or walls) and the (tubular) heating element may extend around at least a portion of the wall(s). In this way, the wall may be located between the inner surface of the heating element and an outer surface of the aerosol-forming article. The wall (or walls) of the cavity may be formed from a thermally conductive material (e.g. a metal) to allow heat conduction from the heating element to the aerosol-forming article. Thus, heat may be conducted from the heating element, through the cavity wall (or walls), to the aerosol-forming substrate of an aerosol-forming article received in the cavity.

In some embodiments the heater may form part of an aerosol-forming article for use with the device. In such cases the device may not comprise a heater. Rather, the aerosol-forming article may comprise a heater. Such arrangements may, for example, be suited to e-cigarette systems in which the aerosol-forming article comprises a tank containing an aerosol former (e.g. in liquid form). In such embodiments, the device may comprise means for connecting the device the heater of an aerosol-forming article engaged with the device. For example, the device may comprise one or more device connectors for (e.g. electrically) connecting the device to a corresponding heater connector of the aerosol-forming article. The connectors (i.e. of both the device and the aerosol-forming article) may be in the form of electrically conductive elements (e.g. plates) that contact when the aerosol-forming article is engaged with the device.

As discussed above the device may comprise a cap disposed at the end of the body that is configured for engagement with an aerosol-forming article. The cap may be moveable between an open position in which access is provided to the heating element, and a closed position in which the cap at least partially encloses the heating element. The cap sensor may be configured to detect whether the cap is in the open position or in the closed position (or between the open and closed positions). The cap may be slideably engaged with the body of the device, and may be slideable between the open and closed positions.

The cap may define at least a portion of the cavity of the device. That is, the cavity may be fully defined by the cap, or each of the cap and body may define a portion of the cavity. The cap may comprise an opening to the cavity. The opening may be configured for receipt of at least a portion of an aerosol-forming article. That is, an aerosol-forming article may be inserted through the opening and into the cavity (so as to be engaged with the device).

The cap may be configured such that when an aerosol-forming article is engaged with the device (e.g. received in the cavity), only a portion of the aerosol-forming article is received in the cavity. That is, a portion of the aerosol-forming article (not received in the cavity) may protrude from (i.e. extend beyond) the opening. This (protruding) portion of the aerosol-forming article may be a terminal (e.g. mouth) end of the aerosol-forming article, which may be received in a user's mouth for the purpose of inhaling aerosol formed by the device.

The device may comprise a power source or may be connectable to a power source (e.g. a power source separate to the device). The power source may be electrically connectable to the heater. In that respect, altering (e.g. toggling) the electrical connection of the power source to the heater may affect a state of the heater. For example, toggling the electrical connection of the power source to the heater may toggle the heater between an on state and an off state. The controller may, for example, prevent supply of power from the power source to the heater when in the safety mode. The power source may be a power store. For example, the power source may be a battery or rechargeable battery (e.g. a lithium ion battery).

The device may comprise an input connection (e.g. a USB port, Micro USB port, USB-C port, etc.). The input connection may be configured for connection to an external source of electrical power, such as a mains electrical supply outlet. The input connection may, in some cases, be used as a substitute for an internal power source (e.g. battery or rechargeable battery). That is, the input connection may be electrically connectable to the heater (for providing power to the heater). Hence, in some forms, the input connection may form at least part of the power source of the device.

Where the power source comprises a rechargeable power source (such as a rechargeable battery), the input connection may be used to charge and recharge the power source.

The device may comprise a user interface (UI), which may comprise the user input module. The user input module of the UI may allow the user to control at least one aspect of the operation of the device. The input module may comprise a power button to switch the device between an on state and an off state. The button may be the same button used to enter safety mode.

In some embodiments the UI may additionally or alternatively comprise an output module to convey information to the user. In some embodiments the output module may comprise a light to indicate a condition of the device (and/or the aerosol-forming article) to the user. The condition of the device (and/or aerosol-forming article) indicated to the user may comprise a condition indicative of the operation of the heater. For example, the condition may comprise whether the heater is in an off state or an on state or whether the device is in safety mode. In some embodiments, the UI unit may comprise at least one of a button, a display, a touchscreen, a switch, a light, and the like. For example, the output module may comprise one or more (e.g. two, three, four, etc.) light-emitting diodes ("LEDs") that may be located on the body of the device. Each of these may be configured to indicate (e.g. when controlled by the controller) that the device is in safety mode.

As above, the device may further comprise a puff sensor (e.g. airflow sensor), which may form part of the input module of the UI. The puff sensor may be configured to detect a user drawing on an end (i.e. a terminal (mouth) end) of the aerosol-forming article. The puff sensor may be configured to produce a signal indicative of a puff state. The signal may be indicative of the user drawing (an aerosol from the aerosol-forming article) such that it is e.g. in the form of a binary signal. Alternatively or additionally, the signal may be indicative of a characteristic of the draw (e.g. a flow rate of the draw, length of time of the draw, etc).

The controller may comprise a microcontroller that may e.g. be mounted on a printed circuit board (PCB). The controller may also comprise a memory, e.g. non-volatile memory. The memory may include instructions, which, when implemented, may cause the controller to perform certain tasks or steps of a method. Where the device comprises an input connection, the controller may be connected to the input connection. The memory may store a state of the device. For example, the memory may store data indicative of whether the device is in a safety mode.

The controller may be configured to control the operation of the heater (and e.g. the heating element). Thus, the controller may be configured to control vaporisation of an aerosol forming part of an aerosol-forming article engaged with the device. The controller may be configured to control the voltage applied by power source to the heater. For example, the controller may be configured to toggle between applying a full output voltage (of the power source) to the heater and applying no voltage to the heater. Alternatively or additionally, the control unit may implement a more complex heater control protocol.

The device may further comprise a voltage regulator to regulate the output voltage supplied by the power source to form a regulated voltage. The regulated voltage may subsequently be applied to the heater.

The controller may be configured to send output signals to a component of the UI. The UI may be configured to convey information to a user, via an output means, in response to such output signals (received from the controller). For example, where the device comprises one or more LEDs, the LEDs may be operatively connected to the controller. Hence, the controller may be configured to control the illumination of the LEDs (e.g. in response to an output signal). For example, the controller may be configured to control the illumination of the LEDs according to (e.g. an on or off) state of the heater.

The device may comprise a wireless interface configured to communicate wirelessly (e.g. via Bluetooth (e.g. a Bluetooth low-energy connection) or Wi-Fi) with an external device. Similarly, the input connection may be configured for wired connection to an external device so as to provide communication between the device and the external device. The command may be in the form of a signal received form the external device.

The external device may be a mobile device. For example, the external device may be a smart phone, tablet, smart watch, or smart car. An application (e.g. app) may be installed on the external device (e.g. mobile device). The application may facilitate communication between the device and the external device via the wired or wireless connection.

The wireless or wired interface may be configured to transfer signals between the external device and the controller of the device. In this respect, the controller may control an aspect of the device in response to a signal received from an external device (e.g. the controller may enter safety mode in response to a signal received from the external device). Alternatively or additionally, an external device may respond to a signal received from the device (e.g. from the controller of the device).

In a second aspect there is provided a smoking substitute system comprising a device according to the first aspect described above, and an aerosol-forming substrate for engagement with the device.

The aerosol-forming article may comprise an aerosol-forming substrate at an upstream end of the aerosol-forming article. The article may be in the form of a smoking substitute article, e.g. heated tobacco (HT) consumable (also known as a heat-not-burn (HNB) consumable).

As used herein, the terms "upstream" and "downstream" are intended to refer to the flow direction of the vapour/aerosol i.e. with the downstream end of the article/consumable being the mouth end or outlet where the aerosol exits the consumable for inhalation by the user. The upstream end of the article/consumable is the opposing end to the downstream end.

The aerosol-forming substrate is capable of being heated to release at least one volatile compound that can form an aerosol. The aerosol-forming substrate may be located at the upstream end of the article/consumable.

In order to generate an aerosol, the aerosol-forming substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The aerosol-forming substrate may comprise plant material. The plant material may comprise least one plant material selected from the list including *Amaranthus dubius, Arctostaphylos uva-ursi* (Bearberry), *Argemone mexicana, Amica*, *Artemisia vulgaris*, Yellow Tees, *Galea zacatechichi*, *Canavalia maritima* (Baybean), *Cecropia mexicana* (Guamura), *Cestrum noctumum*, *Cynoglossum virginianum* (wild comfrey), *Cytisus scoparius*, *Damiana, Entada rheedii*, *Eschscholzia califomica* (California Poppy), *Fittonia albivenis*, *Hippobroma longiflora*, *Humulus japonica* (Japanese Hops), *Humulus lupulus* (Hops), *Lactuca virosa* (Lettuce Opium), *Laggera alata*, *Leonotis leonurus*, *Leonurus cardiaca* (Motherwort), *Leonurus sibiricus* (Honeyweed), *Lobelia cardinalis*, *Lobelia inflata* (Indian-tobacco), *Lobelia siphilitica*, *Nepeta cataria* (Catnip), *Nicotiana species* (Tobacco), *Nymphaea alba* (White Lily), *Nymphaea caerulea* (Blue Lily), Opium poppy, *Passiflora incarnata* (Passionflower), *Pedicularis densiflora* (Indian Warrior), *Pedicularis groenlandica* (Elephant's Head), *Salvia divinorum*, *Salvia dorrii* (Tobacco Sage), Salvia species (Sage), *Scutellaria galericulata*, *Scutellaria lateriflora*, *Scutellaria nana*, *Scutellaria* species (Skullcap), *Sida acuta* (Wireweed), *Sida rhombifolia*, *Silene capensis*, *Syzygium aromaticum* (Clove), *Tagetes lucida* (Mexican Tarragon), *Tarchonanthus camphoratus*, *Tumera diffusa* (Damiana), *Verbascum* (Mullein), *Zamia latifolia* (Maconha Brava) together with any combinations, functional equivalents to, and/or synthetic alternatives of the foregoing.

The plant material may be tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise a gathered sheet of homogenised (e.g. paper/slurry recon) tobacco or gathered shreds/strips formed from such a sheet.

The aerosol-forming substrate may comprise one or more additives selected from humectants, flavourants, fillers, aqueous/non-aqueous solvents and binders.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate may be formed in a substantially cylindrical shape such that the article/consumable resembles a conventional cigarette. It may have a diameter of between 5 and 10mm e.g. between 6 and 9mm or 6 and 8mm e.g. around 7 mm. It may have an axial length of between 10 and 15mm e.g. between 11 and 14mm such as around 12 or 13mm.

The article/consumable may comprise at least one filter element. There may be a terminal filter element at the downstream/mouth end of the article/consumable.

The or at least one of the filter element(s) (e.g. the terminal filter element) may be comprised of cellulose acetate or polypropylene tow. The at least one filter element (e.g. the terminal filter element) may be comprised of activated charcoal. The at least one filter element (e.g. the terminal element) may be comprised of paper. The or each filter element may be at least partly (e.g. entirely) circumscribed with a plug wrap e.g. a paper plug wrap.

The terminal filter element (at the downstream end of the article/consumable) may be joined to the upstream elements forming the article/consumable by a circumscribing tipping layer e.g. a tipping paper layer. The tipping paper may have an axial length longer than the axial length of the terminal filter element such that the tipping paper completely circumscribes the terminal filter element plus the wrapping layer surrounding any adjacent upstream element.

In some embodiments, the article/consumable may comprise an aerosol-cooling element which is adapted to cool the aerosol generated from the aerosol-forming substrate (by heat exchange) before being inhaled by the user.

The article/consumable may comprise a spacer element that defines a space or cavity between the aerosol-forming substrate and the downstream end of the consumable. The spacer element may comprise a cardboard tube. The spacer element may be circumscribed by the (paper) wrapping layer.

In some embodiments the system may be in the form of an e-cigarette system (i.e. rather than a heated tobacco system as described above). In such a system, the consumable may be in the form of an e-cigarette consumable. The e-cigarette system may be configured such that the consumable can be received and retained in the cavity of the device (i.e. so as to be engaged with the device). The consumable may be retained by way of e.g. an interference fit, screwing one onto (or onto) the other, a bayonet fitting, or by way of a snap engagement mechanism.

The consumable may comprise a tank, which may define a reservoir for the storage of an aerosol former. The aerosol former may be in the form of an e-liquid (stored in the reservoir).

The consumable may be a "single-use" consumable. That is, upon exhausting the e-liquid in the tank, the intention may be that the user disposes of the entire consumable. Alternatively, the e-liquid may be the only part of the system that is truly "single-use". For example, the tank may be refillable with e-liquid or another component of the system (internal to the device or external to the device e.g. a refillable cartomizer) may define a reservoir for the e-liquid.

As set forth above, the consumable may comprise a heater (i.e. instead of the heater forming part of the device) configured to heat and vaporise the e-liquid. The consumable may comprise a porous wick that conveys e-liquid from the tank to a heating element of the heater. The heating element may be a heating filament that is wound (e.g. helically) around at least a portion of the porous wick, such that when the heating element is heated (e.g. by the action of electrical current passing through the heating element), heat may be transferred from the heating element to the e-liquid conveyed by the wick. This transfer of heat may vaporise the e-liquid and the resultant vapour may be entrained in an airflow passing through the consumable.

The consumable may further comprise one or more heater connectors for connecting the heater (of the consumable) to the device. The heater connectors may be in the form of electrically conductive element or contacts (e.g. metal plates) and may be disposed on an in-use device-facing surface of the consumable. The heater connectors may be electrically connected to the heater of the consumable, such that electricity supplied via the heater connectors may pass to the heater. In other words, a voltage applied across the heater connectors may generally correspond to a voltage applied across the heating element of the heater.

The heater connectors may be arranged such that they contact corresponding device connectors of the device when the consumable is engaged with the device. The device connectors may be connected (e.g. electrically) to a power source (e.g. battery) of the device. Thus, electricity may be supplied from the power source to the heating element, via in-contact heater and device connectors. In this way, the heater forming part of the consumable may operate (and interact with e.g. a controller) as otherwise described above with respect to a heater forming part of the device.

According to a third aspect, there is provided a method of operating a smoking substitute device, the method comprising: receiving a command to enter a safety mode; and disabling at least one function of the device in response to the command.

Disabling at least one function of the device comprises disabling the operation one or more of a heater, display, light indicator, battery level indicator, and a haptic feedback module of the device.

The method may comprise transmitting the command in response to user input. The user input may be as set forth above with respect to the first aspect. For example, the user input may comprise a pattern of button presses.

The method may comprise transmitting the command in response to an operating condition of the device. The operating condition may be as described above with respect to the first aspect.

The method may comprise enabling the at least one function of the device in response to a command to leave the safety mode. The command to leave the safety mode may be as described above with respect to the first aspect. For example, the command may be the same as the command to enter safety mode. In this respect, the command may toggle safety mode (on and off).

According to a fourth aspect there is provided a method of operating a smoking substitute device, the method comprising entering the device into a safety mode (e.g. child safety mode) based upon a user input to a user interface of the device or based upon a pre-determined operating condition of the device being met.

The method of the fourth aspect may be as otherwise described with respect to the third aspect.

According to a fifth aspect, there is provided a smoking substitute device comprising controller configured to enter a safety mode (e.g. child safety mode) based on a user input to a user interface of the device or based upon a predetermined condition of the device being met.

The device of the fifth aspect may be as otherwise described with respect to the first aspect.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### SUMMARY OF THE FIGURES

So that the invention may be understood, and so that further aspects and features thereof may be appreciated, embodiments illustrating the principles of the invention will now be discussed in further detail with reference to the accompanying figures, in which:
Figure 1A is a schematic of a smoking substitute system;
Figure 1B is a schematic of a variation of the smoking substitute system of Figure 1A;
Figure 2A is a front view of a first embodiment of a smoking substitute system with the consumable engaged with the device;
Figure 2B is a front view of the first embodiment of the smoking substitute system with the consumable disengaged from the device;
Figure 2C is a section view of the consumable of the first embodiment of the smoking substitute system;
Figure 2D is a detailed view of an end of the device of the first embodiment of the smoking substitute system;
Figure 2E is a section view of the first embodiment of the substitute smoking system;
Figure 3 is a flowchart illustrating method of operating a device in a safety mode;
Figure 4A is a front view of a second embodiment of a smoking substitute system with the consumable engaged with the device; and
Figure 4B is a front view of a second embodiment of the smoking substitute system with the consumable disengaged from the device.

### DETAILED DESCRIPTION OF THE INVENTION

Aspects and embodiments of the present invention will now be discussed with reference to the accompanying figures. Further aspects and embodiments will be apparent to those skilled in the art. All documents mentioned in this text are incorporated herein by reference.

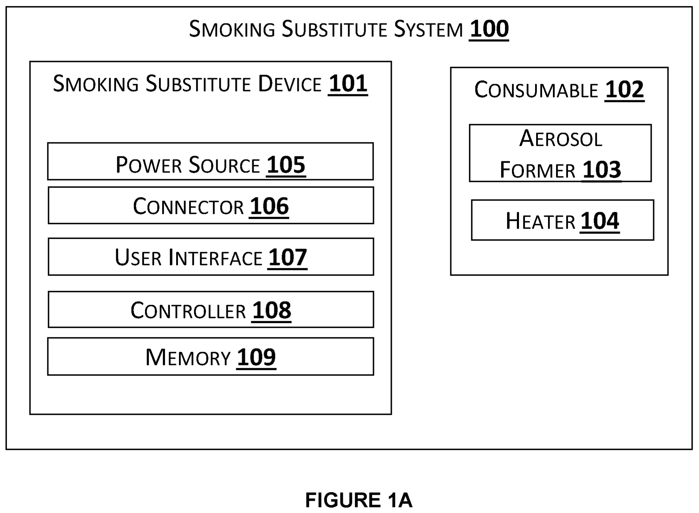

Figure 1A is a schematic providing a general overview of a smoking substitute system 100. The system 100 includes a substitute smoking device 101 and an aerosol-forming article in the form of a consumable 102, which comprises an aerosol former 103. The system is configured to vaporise the aerosol former by heating the aerosol former 103 (so as to form a vapour/aerosol for inhalation by a user).

In the illustrated system, the heater 104 forms part of the consumable 102 and is configured to heat the aerosol former 103. Heat from the heater 104 vaporises the aerosol former 103 to produce a vapour. The vapour subsequently condenses to form an aerosol, which is ultimately inhaled by the user.

The system 100 further comprises a power source 105 that forms part of the device 101. In other embodiments the power source 105 may be external to (but connectable to) the device 101. The power source 105 is electrically connectable to the heater 104 such that the power source 105 is able to supply power to the heater 104 (i.e. for the purpose of heating the aerosol former 103). Thus, control of the electrical connection of the power source 105 to the heater 104 provides control of the state of the heater 104. The power source 105 may be a power store, for example a battery or rechargeable battery (e.g. a lithium ion battery).

The system 100 further comprises an I/O module comprising a connector 106 (e.g. in the form of a USB port, Micro USB port, USB-C port, etc.). The connector 106 is configured for connection to an external source of electrical power, e.g. a mains electrical supply outlet. The connector 106 may be used in substitution for the power source 105. That is the connector 106 may be electrically connectable to the heater 104 so as to supply electricity to the heater 104. In such embodiments, the device may not include a power source, and the power source of the system may instead comprise the connector 106 and an external source of electrical power (to which the connector 106 provides electrical connection).

In some embodiments, the connector 106 may be used to charge and recharge the power source 105 where the power source 104 includes a rechargeable battery.

The system 100 also comprises a user interface (UI) 107. Although not shown, the UI 107 may include input means to receive commands from a user. The input means of the UI 107 allows the user to control at least one aspect of the operation of the system 100. The input means may, for example, be in the form of a button, touchscreen, switch, microphone, etc.

The UI 107 also comprises output means to convey information to the user. The output means may, for example, comprise lights (e.g. LEDs), a display screen, speaker, vibration generator, etc.

The system 100 further comprises a controller 108 and a memory 109 coupled to the controller 108. In the illustrated embodiment, the controller 108 is a component of the device 101, but in other embodiments may be separate from (but connectable to) the device 101. The controller 108 is configured to control at least one function of the device 101. The memory 109 stores controller-executable instructions that causes the controller 109 to perform one or more functions. The controller 108 is configured to control the operation of the heater 104 and, for example, may be configured to control the voltage applied from the power source 105 to the heater 104. The controller 108 may be configured to toggle the supply of power to the heater 105 between an on state, in which the full output voltage of the power source 105 is applied to the heater 104, and an off state, in which the no voltage is applied to the heater 104.

Although not shown, the system 100 may also comprise a voltage regulator to regulate the output voltage from the power source 105 to form a regulated voltage. The regulated voltage may then be applied to the heater 104.

In addition to being connected to the heater 104, the controller 108 is operatively connected to the UI 107. Thus, the controller 108 may receive an input signal from the input means of the UI 107. Similarly, the controller 108 may transmit output signals to the UI 107. In response, the output means of the UI 107 may convey information, based on the output signals, to a user.

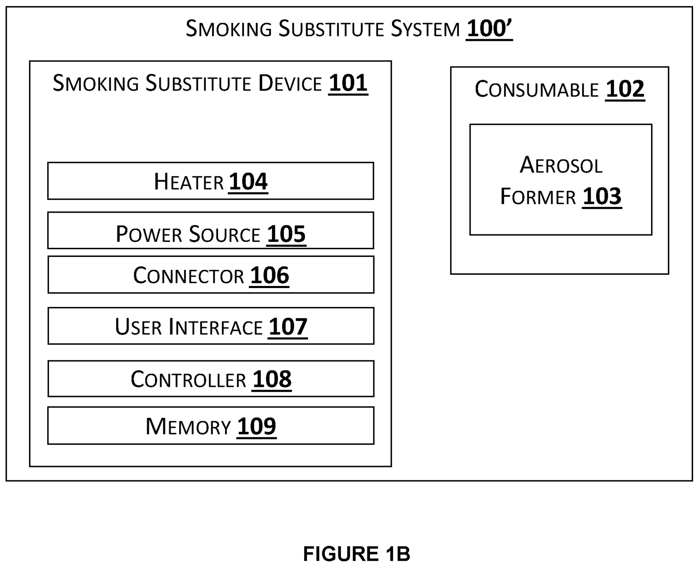

Figure 1B is a schematic showing a variation of the system 100 of Figure 1A. In the system 100' of Figure 1B, the heater 104 forms part of the device 101, rather than the consumable 102. In this variation, the heater 104 is electrically connected to the power source 105.

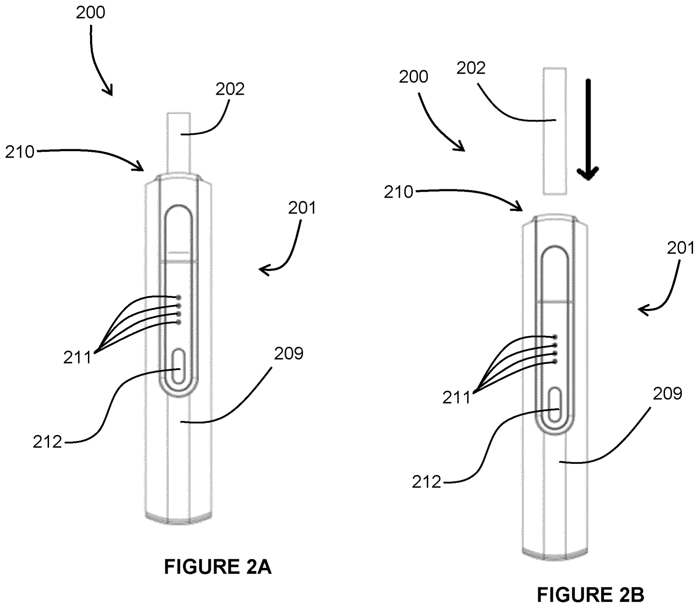

Figures 2A and 2B illustrate a heated-tobacco (HT) smoking substitute system 200. The system 200 is an example of the systems 100, 100' described in relation to Figures 1A or 1B. System 200 includes an HT device 201 and an HT consumable 202. The description of Figures 1A and 1B above is applicable to the system 200 of Figures 2A and 2B, and will thus not be repeated.

The device 201 and the consumable 202 are configured such that the consumable 202 can be engaged with the device 201. Figure 2A shows the device 201 and the consumable 202 in an engaged state, whilst Figure 2B shows the device 201 and the consumable 202 in a disengaged state.

The device 201 comprises a body 209 and cap 210. In use the cap 209 is engaged at an end of the body 209. Although not apparent from the figures, the cap 210 is moveable relative to the body 209. In particular, the cap 210 is slideable and can slide along a longitudinal axis of the body 209.

The device 201 comprises an output module (forming part of the UI of the device 201) in the form of a plurality of light-emitting diodes (LEDs) 211 arranged linearly along the longitudinal axis of the device 201 and on an outer surface of the body 209 of the device 201. A button 212 is also arranged on an outer surface of the body 209 of the device 201 and is axially spaced (i.e. along the longitudinal axis) from the plurality of LEDs 211.

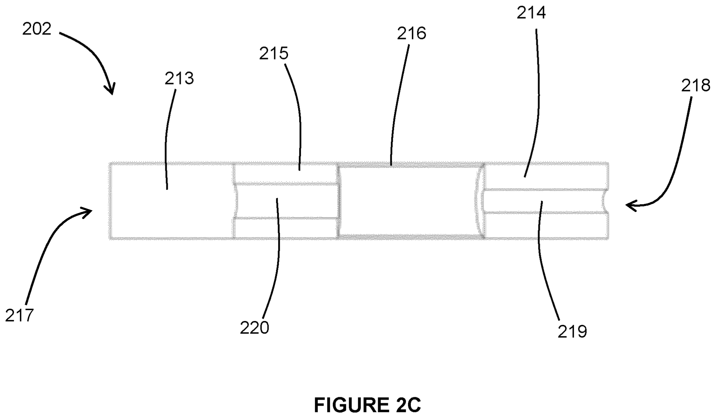

Figure 2C show a detailed section view of the consumable of 202 of the system 200. The consumable 202 generally resembles a cigarette. In that respect, the consumable 202 has a generally cylindrical form with a diameter of 7 mm and an axial length of 70 mm. The consumable 202 comprises an aerosol forming substrate 213, a terminal filter element 214, an upstream filter element 215 and a spacer element 216. In other embodiments, the consumable may further comprise a cooling element. A cooling element may exchange heat with vapour that is formed by the aerosol-forming substrate 213 in order to cool the vapour so as to facilitate condensation of the vapour.

The aerosol-forming substrate 213 is substantially cylindrical and is located at an upstream end 217 of the consumable 202, and comprises the aerosol former of the system 200. In that respect, the aerosol forming substrate 213 is configured to be heated by the device 201 to release a vapour. The released vapour is subsequently entrained in an airflow flowing through the aerosol-forming substrate 213. The airflow is produced by the action of the user drawing on a downstream 218 (i.e. terminal or mouth end) of the consumable 202.

In the present embodiment, the aerosol forming substrate 213 comprises tobacco material that may, for example, include any suitable parts of the tobacco plant (e.g. leaves, stems, roots, bark, seeds and flowers). The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon). For example, the aerosol-forming substrate 213 may comprise a gathered sheet of homogenised (e.g. paper/slurry recon) tobacco or gathered shreds/strips formed from such a sheet.

In order to generate an aerosol, the aerosol forming substrate 213 comprises at least one volatile compound that is intended to be vaporised/aerosolised and that may provide the user with a recreational and/or medicinal effect when inhaled. The aerosol-forming substrate 213 may further comprise one or more additives. For example, such additives may be in the form of humectants (e.g. propylene glycol and/or vegetable glycerine), flavourants, fillers, aqueous/non-aqueous solvents and/or binders.

The terminal filter element 214 is also substantially cylindrical, and is located downstream of the aerosol forming substrate 213 at the downstream end 218 of the consumable 202. The terminal filter element 214 is in the form of a hollow bore filter element having a bore 219 (e.g. for airflow) formed therethrough. The diameter of the bore 219 is 2 mm. The terminal filter element 214 is formed of a porous (e.g. monoacetate) filter material. As set forth above, the downstream end 218 of the consumable 202 (i.e. where the terminal filter 214 is located) forms a mouthpiece portion of the consumable 202 upon which the user draws. Airflow is drawn from the upstream end 217, thorough the components of the consumable 202, and out of the downstream end 218. The airflow is driven by the user drawing on the downstream end 218 (i.e. the mouthpiece portion) of the consumable 202.

The upstream filter element 215 is located axially adjacent to the aerosol-forming substrate 213, between the aerosol-forming substrate 213 and the terminal filter element 214. Like the terminal filter 214, the upstream filter element 215 is in the form of a hollow bore filter element, such that it has a bore 220 extending axially therethrough. In this way, the upstream filter 215 may act as an airflow restrictor. The upstream filter element 215 is formed of a porous (e.g. monoacetate) filter material. The bore 220 of the upstream filter element 214 has a larger diameter (3 mm) than the terminal filter element 214.

The spacer 216 is in the form of a cardboard tube, which defines a cavity or chamber between the upstream filter element 215 and the terminal filter element 214. The spacer 216 acts to allow both cooling and mixing of the vapour/aerosol from the aerosol-forming substrate 213. The spacer has an external diameter of 7 mm and an axial length of 14mm.

Although not apparent from the figure, the aerosol-forming substrate 213, upstream filter 215 and spacer 216 are circumscribed by a paper wrapping layer. The terminal filter 214 is circumscribed by a tipping layer that also circumscribes a portion of the paper wrapping layer (so as to connect the terminal filter 214 to the remaining components of the consumable 202). The upstream filter 215 and terminal filter 214 are circumscribed by further wrapping layers in the form of plug wraps.

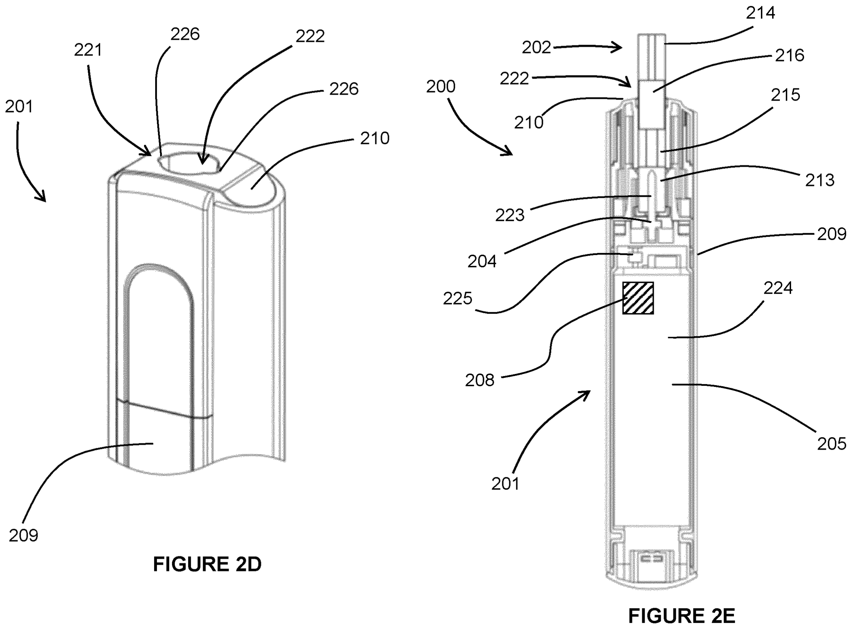

Returning now to the device 201, Figure 2D illustrates a detailed view of the end of the device 201 that is configured to engage with the consumable 202. The cap 210 of the device 201 includes an opening 221 to an internal cavity 222 (more apparent from Figure 2D) defined by the cap 210. The opening 221 and the cavity 222 are formed so as to receive at least a portion of the consumable 202. During engagement of the consumable 202 with the device 201, a portion of the consumable 202 is received through the opening 221 and into the cavity 222. After engagement (see Figure 2B), the downstream end 218 of the consumable 202 protrudes from the opening 221 and thus protrudes also from the device 201. The opening 221 includes laterally disposed notches 226. When a consumable 202 is received in the opening 221, these notches 226 remain open and could, for example, be used for retaining a cover to cover the end of the device 201.

Figure 2E shows a cross section through a central longitudinal plane through the device 201. The device 201 is shown with the consumable 202 engaged therewith.

The device 201 comprises a heater 204 comprising heating element 223. The heater 204 forms part of the body 209 of the device 201 and is rigidly mounted to the body 209. In the illustrated embodiment, the heater 204 is a rod heater with a heating element 223 having a circular transverse profile. In other embodiments the heater may be in the form of a blade heater (e.g. heating element with a rectangular transverse profile) or a tube heater (e.g. heating element with a tubular form).

The heating element 223 of the heater 204 projects from an internal base of the cavity 222 along a longitudinal axis towards the opening 221. As is apparent from the figure, the length (i.e. along the longitudinal axis) of the heating element is less than a depth of the cavity 222. In this way, the heating element 223 does not protrude from or extend beyond the opening 221.

When the consumable 202 is received in the cavity 222 (as is shown in Figure 2D), the heating element 223 penetrates the aerosol-forming substrate 213 of the consumable 202. In particular, the heating element 223 extends for nearly the entire axial length of the aerosol-forming substrate 213 when inserted therein. Thus, when the heater 204 is activated, heat is transferred radially from an outer circumferential surface the heating element 223 to the aerosol-forming substrate 213.

The device 202 further comprises an electronics cavity 224. A power source, in the form of a rechargeable battery 205 (a lithium ion battery), is located in electronics cavity 224.

The device 202 includes a connector (i.e. forming part of an IO module of the device 201) in the form of a USB port 206. The connector may alternatively be, for example, a micro-USB port or a USB-C port for examples. The USB port 206 may be used to recharge the rechargeable battery 205.

The device 202 includes a controller 208 located in the electronics cavity 224. The controller comprises a microcontroller mounted on a printed circuit board (PCB). The USB port 206 is also connected to the controller 208 (i.e. connected to the PCB and microcontroller).

As will be described in more detail below, the controller 208 is configured (among other functions) to enter a safety mode in response to a command. In the illustrated embodiment, this command is in the form of a series of presses (e.g. five successive presses) of the button 212. When the controller 208 enters safety mode, the LEDs 111 flash to indicate that safety mode has been entered. In safety mode, the controller 208 disables the heater 204 of the device 201, such that subsequent button presses (which would normally activate the heater 204) do not activate the heater 204. Instead, these subsequent button presses cause the controller 208 to control a vibration mechanism(not shown) of the device 201 to vibrate so as to provide haptic feedback to a user holding the device 201. The controller 208 is further configured to leave safety mode when it receives a leave command in the form of a further series of button presses. Upon leaving safety mode, the controller enables the heater 204, such that a further button press activates the heater 204.

The controller 208 is also configured to enter safety mode when the cap 210 of the device 209 is slid from a closed position to an open position. Although not apparent from the figures, other operating conditions may also cause the controller 208 to enter safety mode, such as ambient temperature exceeding threshold temperature, overheating of the battery 205 or the heating element 223, or non-usage of the device 201 for a predefined time period.

The controller 208 is configured to control further functions of the device 202. For example, the controller 208 is configured to control the operation of the heater 204. Such control of the operation of the heater 204 may be accomplished by the controller toggling the electrical connection of the rechargeable battery 205 to the heater 204. For example, the controller 208 is configured to control the heater 204 in response to first user input received via the UI of the device 201. The first user input may be for example, includes one of predetermined sequence of presses of the button 212 and combination of long and short button press sequence. The controller 208 receives the first user input and determines the validity of the first user input based on comparison of the first user input with a predetermined locking key. The controller 208 enables the user to previously set the predetermined locking key via the UI of the device 201.

The device 202 comprises a further input means (i.e. in addition to the button 212) in the form of a puff sensor 225. The puff sensor 225 is configured to detect a user drawing (i.e. inhaling) at the downstream end 218 of the consumable 202. The puff sensor 225 may, for example, be in the form of a pressure sensor, flowmeter or a microphone. The puff sensor 225 is operatively connected to the controller 208 in the electronics cavity 224, such that a signal from the puff sensor 225, indicative of a puff state (i.e. drawing or not drawing), forms an input to the controller 208 (and can thus be responded to by the controller 208).

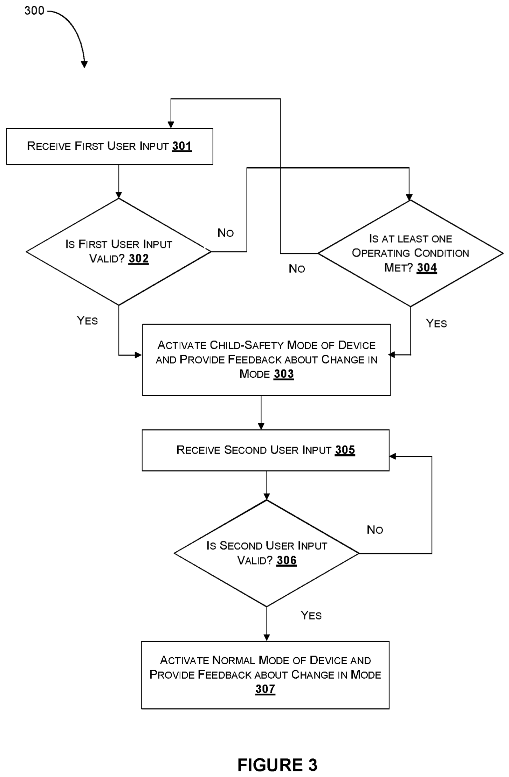

Figure 3 illustrates a flowchart providing an exemplary method of operating a device (such as a device similar to those described above and below) in child-safety mode when configured.

As illustrated in Figure 3, the method 300 includes one or more blocks implemented by a controller of a device. The method 300 may be described in the general context of controller executable instructions. Generally, controller executable instructions can include routines, programs, objects, components, data structures, procedures, modules, and functions, which perform particular functions or implement particular abstract data types.

The order in which the method 300 is described is not intended to be construed as a limitation, and any number of the described method blocks can be combined in any order to implement the method 300. Additionally, individual blocks may be deleted from the method 300 without departing from the scope of the subject matter described herein. Furthermore, the method 300 can be implemented in any suitable hardware, software, firmware, or combination thereof.

At block 301, the controller receives the first user input via a UI of the device. The first user input includes one of predetermined sequence of presses of at least one button and combination of long and short button press sequence.

At block 302, the controller validates the first user input. The controller validates the first user input by comparing the first user input with a predetermined sequence of presses for entering a safety mode. If the controller determines the first user input to be a command for entering safety mode (in this case a child safety mode), then the method proceeds to block 303 along the "YES" path. Otherwise, the method proceeds to block 304 along the "NO" path.

At block 303, the controller activates the safety mode of the device and provides feedback indicative of the change in mode. The controller configures the device to enter into the safety mode. For example, the controller may disable the heater or a display of the device. The feedback may be a haptic feedback. In another example, the feedback may be an audio feedback output from a speaker of the device.

At block 304, the controller determines whether at least one operating condition of the device is present. For example, the one or more operating conditions may include the cap being open, ambient temperature exceeding threshold temperature, overheating of battery or heating element, or non-usage of the device for a predefined time period. If the controller 208 determines that at least one of the operating conditions is present, then the method proceeds to block 303 along the "YES" path where the controller activates the safety mode of the device. Alternatively, if the controller determines that none of the operating conditions of the device are met, then the method proceeds to block 301 to receive the first user input, or where the input is e.g. for control of the device, the controller may control the device accordingly.

At block 305, the controller receives a second user input whilst the device 201 is operating in safety mode. This second input may cause the controller to leave the safety mode. The second user input may for example include an inhale pattern measured by a puff sensor of the device.

At block 306, the controller 208 determines the validity of the second user input. The controller compares the second user input with a predetermined unlocking pattern (e.g. pattern of button presses) to determine the validity of the second user input. If the controller determines that the second user input is valid, then the method proceeds to block 307 along the "YES" path; otherwise, the method proceeds to block 305 to receive the second user input along the "NO" path.

At block 307, the controller leaves safety mode and provides feedback regarding this change in mode. In this respect, the controller enables the previously disabled functions of the device. The controller also generates haptic feedback such as one or more vibrations to indicate to the user that the device has left the safety mode.

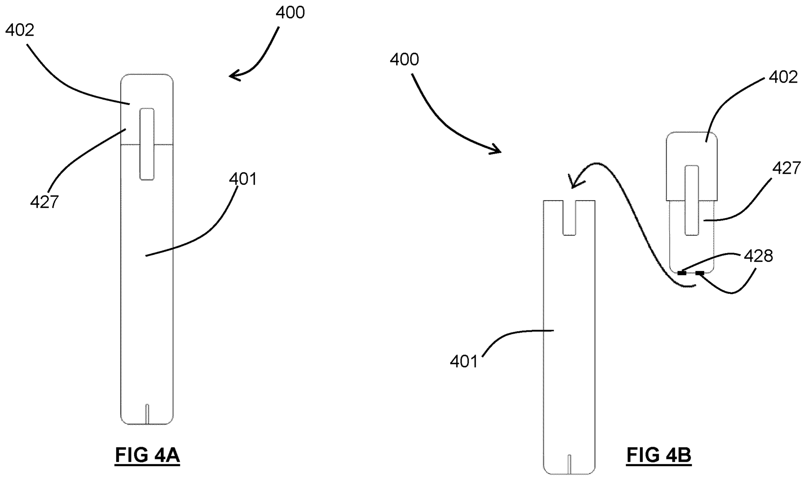

Figures 4A and 4B illustrate an e-cigarette smoking substitute system 400. The system 400 is an example of the systems 100, 100' of Figures 1A and 1B and comprises an e-cigarette device 401 and an e-cigarette consumable 402. The description of Figures 1A and 1B above is applicable to the system of Figures 4A and 4B, and will not be repeated. It should be appreciated that this system 400 may be controlled in the same manner as the system described above with respect to Figure 2A to 2E. That is, the system 400 may comprise a controller configured to enter a safety mode upon receipt of a command as is described above.

The device 401 and the consumable 402 are configured such that the consumable 402 can be engaged with the device 401. Figure 4A shows the device 401 and the consumable 402 in an engaged state, whilst Figure 4B shows the device 401 and the consumable 402 in a disengaged state. During engagement a portion of the consumable 402 is received in a cavity 422 of the device 401. The consumable 402 is retained in the device 401 via an interference fit (although in other embodiments, the device and consumable could be engaged by screwing one onto (or onto) the other, through a bayonet fitting, or by way of a snap engagement mechanism).

The consumable 402 includes a tank 427. The tank 427 defines a reservoir for the storage of an aerosol-former, which in this embodiment, is in the form of e-liquid.

In this present embodiment, the consumable 402 is a "single-use" consumable. That is, upon exhausting the e-liquid in the tank 427, the intention is that the user disposes of the whole consumable 402. In other embodiments, the e-liquid (i.e. aerosol former) may be the only part of the system that is truly "single-use". In such embodiments, the tank may be refillable with e-liquid or the e-liquid may be stored in a non-consumable component of the system. For example, the e-liquid may be stored in a tank located in the device or stored in another component that is itself not single-use (e.g. a refillable cartomizer).

In the illustrated system 400, a heater 404 is located in the consumable 402 and is configured to heat and vaporise the e-liquid (stored in the tank 427). Although not shown, the heater 404 comprises a porous wick and a resistive heating element. The porous wick conveys e-liquid from the tank 427 to the heating element. The heating element is a heating filament that is helically wound around a portion of the porous wick, such that when the heating element is heated (e.g. by the action of electrical current passing through the heating element), heat is transferred from the heating element to the e-liquid conveyed by the wick. This transfer of heat vaporises the e-liquid and the resultant vapour is entrained in an airflow passing through the consumable 402 (i.e. driven by a user drawing on a downstream end 418 of the consumable 402). Between the vaporisation point at the coil and the downstream end 418 (i.e. the mouth end), the vapour condenses into an aerosol, and is subsequently inhaled by the user.

Like the previously described embodiment, the device 401 comprises a power source in the form of a rechargeable battery (not shown) and a connector in the form of a USB port (not shown). The device 401 further comprises controller (also not shown). The rechargeable battery, connector and controller are similar (and operate in a similar manner) to the corresponding components of the embodiment described above with respect to Figure 2A to 2E.

The consumable 402 includes a pair of heater electrical contacts 428 disposed on a device-facing end surface of the consumable 402. The heater electrical contacts 428 are electrically connected to the heater 404 in the consumable 402, such that a voltage applied across the heater electrical contacts 428 generally corresponds to a voltage applied across the resistive heating element of the heater 404.

When the consumable 402 is engaged with the device 401, the heater electrical contacts 428 are brought into electrical contact with corresponding device electrical contacts (not shown) on the device 401. The device electrical contacts are electrically connected (directly or indirectly) to the rechargeable battery. The controller may thus be configured to control the voltage applied across the device electrical contacts from the rechargeable battery. By controlling the voltage applied across the device electrical contacts, the voltage applied to the heater 404 is correspondingly controlled.

The device 401 includes an output means (forming part of the UI of the system 300) in the form of a single light-emitting diode ("LED") 411. The LED 411 is operatively connected to the controller, such that controller can control the illumination of the LED 411. The controller is configured to illuminate the LED when then the heater 404 is active.

The device 401 also includes an input means in the form of a puff sensor (not shown). The puff sensor is the same as that described above with respect to the embodiment shown in Figure 2A to 2E.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

Features of embodiments of the invention are set out in the following paragraphs.

Clause 1. A smoking substitute device comprising a controller configured to receive a command to enter a safety mode and, in response, disable at least one function of the device.

Clause 2. A device according to clause 1, wherein disabling at least one function of the device comprises disabling the operation one or more of a heater, user input, display, light indicator, battery level indicator, and a haptic feedback module of the device.

Clause 3. A device according to clause 1 or 2, wherein the device comprises a user input module operatively connected to the controller, the user input module configured to transmit the command to enter the safety mode to the controller based an interaction by a user with the user input module.

Clause 4. A device according to clause 3 wherein the user input module comprises a button and wherein the command comprises a signal indicative of a pattern of button presses.

Clause 5. A device according to clause 3 or 4 wherein the user input module comprises a button and wherein the command comprises a signal indicative of the button being depressed for a predetermined time period.

Clause 6. A device according to clause 1 or 2 wherein the command is generated in response to an operating condition of the device.

Clause 7. A device according to clauses 6 wherein the device comprises a heater, a body, a cap engagable with the body for at least partly enclosing the heater, and a cap sensor for detecting whether the cap is open, the command comprising a signal from the cap sensor indicative of the cap being open.

Clause 8. A device according to any one of the preceding clauses wherein the controller is configured to enable the at least one function of the device upon receipt of a command to leave the safety mode.

Clause 9. A device according to any one of the clauses comprising a user output module configured to provide haptic feedback to a user of the device, the controller configured to control the output module to provide haptic feedback when the safety mode is entered.

Clause 10. A method of operating a smoking substitute device, the method comprising: receiving a command to enter a safety mode; and disabling at least one function of the device in response to the command.

Clause 11. A method according to clause 10, wherein disabling at least one function of the device comprises disabling the operation one or more of a heater, display, light indicator, battery level indicator, and a haptic feedback module of the device.

Clause 12. A method according to clause 10 or 11 comprising transmitting the command in response to user input.

Clause 13. A method according to clause 12 wherein the user input comprises a pattern of button presses.

Clause 14. A method according to any one of clauses 10 to 13 comprising transmitting the command in response to an operating condition of the device.

Clause 15. A method according to any one of clauses 10 to 14 comprising enabling the at least one function of the device in response to a command to leave the safety mode.

## Claims

1. A smoking substitute device comprising a controller configured to receive a command to enter a safety mode and, in response, disable at least one function of the device.

2. The smoking substitute device according to claim 1, comprising a user output module.

3. The smoking substitute device according to claim 2, wherein the controller is configured to control the user output module to provide a user output when a user attempts to activate the disabled function while in the safety mode.

4. The smoking substitute device of claim 3, wherein the user's attempt to activate the disabled function is in the form of an inhalation from the device while in the safety mode, the device comprising a sensor for detecting the inhalation.

5. The smoking substitute device according to any one of claims 2 to 4, wherein the user output module is configured to provide haptic feedback and/or wherein the user output module comprises one or more lights.

6. The smoking substitute device according to any one of claims 2 to 5, wherein the controller is configured to control the user output module to provide a user output when the safety mode is entered.

7. The smoking substitute device according to any one of claims 2 to 6, wherein the controller is configured to control the output module to provide a user output when leaving the safety mode.

8. The smoking substitute device according to any preceding claim, wherein disabling at least one function of the device comprises disabling the operation of a heater, and wherein the controller is configured to illuminate one or more lights to indicate whether the heater is in an on state or an off state..

9. The smoking substitute device according to any preceding claim, wherein the device comprises a user input module operatively connected to the controller, the user input module configured to transmit the command to enter the safety mode to the controller based an interaction by a user with the user input module.

10. The smoking substitute device according to any one of the preceding claims, wherein the at least one function of the device that is disabled in safety mode is configurable by a user via interaction with the user input module.

11. The smoking substitute device according to claim 9 or 10, wherein the user input module comprises a button and wherein the command comprises a signal indicative of a pattern of button presses or a signal indicative of the button being depressed for a predetermined time period.

12. The smoking substitute device according to any one of claims 1 to 10, wherein the command is generated in response to an operating condition of the device.

13. The smoking substitute device according to claim 12, wherein the command is generated in response to the absence of a consumable engaged with the device or wherein the command comprises a signal indicative of an absence of user interaction or input for a predetermined time period.

14. The smoking substitute device according to any one of the preceding claims wherein the controller is configured to enable the at least one function of the device upon receipt of a command to leave the safety mode, wherein the command to leave the safety mode is the same as the command to enter the safety mode.

15. A method of operating a smoking substitute device, the method comprising:
receiving a command to enter a safety mode; and
disabling at least one function of the device in response to the command.
